# EUROPEAN PATENT APPLICATION

(11) **EP 4 706 695 A1**
(43) Date of publication of application: **11.03.2026**
(21) Application number: 25191034.5
(22) Date of filing: 22.07.2025
(51) Int. Cl.: A61L 27/30, A61L 27/50

(54) **SPUTTER COATED FABRIC FOR HEART VALVE APPLICATIONS AND METHODS OF MAKING SAME**

(30) Priority: 06.09.2024 US 202463691561 P
(71) Applicant: St. Jude Medical, Cardiology Division, Inc., St. Paul, MN 55117 (US)
(72) Inventor: BARRETTE, Alexander, Maplewood, MN 55109 (US); ASHWORTH, Paul E., Danbury, WI 54830 (US); MINION, William, Vadnais Heights, MN 55109 (US); REIMER, Jay, Shoreview, MN 55126 (US); GREEN, Chad, Forest Lake, MN 55025 (US)
(74) Representative: Gill Jennings & Every LLP

(57) **Abstract**

In some examples, a method of manufacturing a prosthetic heart valve includes providing a stent and a valve assembly, the valve assembly including at least one of an inner cuff and an outer cuff, and a plurality of leaflets, sputter coating a layer of one or more target materials onto a surface of at least one component selected from the plurality of leaflets, the inner cuff and the outer cuff, and coupling the valve assembly to the stent.

## Description

### Cross-Reference to Related Applications

This application claims the benefit of U.S. Provisional Patent Application No. 63/691,561, filed on September 6, 2024, the entire contents of which are hereby incorporated by reference in their entirety.

### Background of the Disclosure

Valvular heart disease, and specifically aortic and mitral valve disease, is a significant health issue in the United States. Valve replacement is one option for treating heart valve diseases. Prosthetic heart valves include surgical heart valves, as well as collapsible and expandable heart valves intended for transcatheter aortic valve replacement or implantation ("TAVR" or "TAVI") or transcatheter mitral valve replacement ("TMVR"). Surgical or mechanical heart valves may be sutured into a native annulus of a patient during an open-heart surgical procedure, for example. Collapsible and expandable heart valves may be delivered into a patient via a delivery apparatus such as a catheter to avoid a more invasive procedure such as full open-chest, open-heart surgery. As used herein, reference to a "collapsible and expandable" heart valve includes heart valves that are formed with a small cross-section that enables them to be delivered into a patient through a catheter in a minimally invasive procedure, and then expanded to an operable state once in place, as well as heart valves that, after construction, are first collapsed to a small cross-section for delivery into a patient and then expanded to an operable size once in place in the valve annulus.

Collapsible and expandable prosthetic heart valves typically take the form of a one-way valve structure (often referred to as a valve assembly) mounted within an expandable frame (the terms "stent" and "frame" may be used interchangeably herein). In general, these collapsible and expandable heart valves include a self-expanding, mechanically-expandable, or balloon-expandable frame, often made of nitinol or another shape-memory metal or metal alloy (for self-expanding frames) or steel or cobalt chromium (for balloon-expandable frames). The one-way valve assembly mounted to/within the stent includes one or more leaflets and may also include a cuff or skirt. The cuff may be disposed on the stent's interior or luminal surface, its exterior or abluminal surface, and/or on both surfaces. A cuff helps to ensure that blood does not just flow around the valve leaflets if the valve or valve assembly is not optimally seated in a valve annulus. A cuff, or a portion of a cuff disposed on the exterior of the stent, can help prevent leakage around the outside of the valve (the latter known as paravalvular or "PV" leakage).

Balloon expandable valves are typically delivered to the native annulus while collapsed (or "crimped") onto a deflated balloon of a balloon catheter, with the collapsed valve being either covered or uncovered by an overlying sheath. Once the crimped prosthetic heart valve is positioned within the annulus of the native heart valve that is being replaced, the balloon is inflated to force the balloon-expandable valve to transition from the collapsed or crimped condition into an expanded or deployed condition, with the prosthetic heart valve tending to remain in the shape into which it is expanded by the balloon. Typically, when the position of the collapsed prosthetic heart valve is determined to be in the desired position relative to the native annulus (e.g. via visualization under fluoroscopy), a fluid (typically a liquid although gas could be used as well) such as saline is pushed via a syringe (manually, automatically, or semi-automatically) through the balloon catheter to cause the balloon to begin to fill and expand, and thus cause the overlying prosthetic heart valve to expand into the native annulus.

Certain materials, such as tissue, degrade over time, and alternative materials, including fabrics, polymers or metals or combinations of the previous may represent an opportunity to improve the durability of a surgical or transcatheter (e.g., self-expanding or balloon expanding) heart valve replacements. However, α-smooth muscle actin (α-SMA) cells are known to have adverse effects (e.g., leaflet retraction) *in vivo,* reducing function of these alternative materials. It would be beneficial to further process certain components of a prosthetic heart valve (or other implants or medical devices) to mitigate cell attachment.

### Summary of the Disclosure

In some examples, a method of manufacturing a prosthetic heart valve includes providing a stent and a valve assembly, the valve assembly including at least one of an inner cuff and an outer cuff, and a plurality of leaflets, sputter coating a layer of one or more target materials onto a surface of at least one component selected from the plurality of leaflets, the inner cuff and the outer cuff, and coupling the valve assembly to the stent.

### Brief Description of the Drawings

Fig. 1 is a perspective view of an example of a prosthetic heart valve.
Fig. 2 is a front view of an example of a section of the frame of the prosthetic heart valve of Fig. 1, as if cut longitudinally and laid flat on a table.
Fig. 3 is a front view of an example of a prosthetic leaflet of the prosthetic heart valve of Fig. 1, as if laid flat on a table.
Fig. 4 is a top view of the prosthetic heart valve of Fig. 1 mounted on an example of a portion of a delivery system.
Fig. 5 is an enlarged view of the handle of the delivery system shown in Fig. 4.
Fig. 6 is an enlarged view of a distal end of the delivery system shown in Fig. 4.
Fig. 7 is a top view of an example of a balloon catheter when the balloon is inflated.
Fig. 8 is a top view of an example of an inflation system for use with a delivery system similar to that shown in Fig. 4.
Fig. 9 is a side view of the inflation system of Fig. 8.
Fig. 10 is a perspective view of a connection between the inflation system of Figs. 8-9 and the handle of the delivery system of Fig. 4.
Fig. 11 is a flowchart showing exemplary steps in a procedure to implant the prosthetic heart valve of Fig. 1 into a patient using the delivery system of Fig. 4.
Fig. 12 is a flowchart showing exemplary steps in a sputter coating procedure.

### Detailed Description of the Disclosure

As used herein, the term "inflow end" when used in connection with a prosthetic heart valve refers to the end of the prosthetic valve into which blood first enters when the prosthetic valve is implanted in an intended position and orientation, while the term "outflow end" refers to the end of the prosthetic valve where blood exits when the prosthetic valve is implanted in the intended position and orientation. Thus, for a prosthetic aortic valve, the inflow end is the end nearer the left ventricle while the outflow end is the end nearer the aorta. The intended position and orientation are used for the convenience of describing valves disclosed herein. However, it should be noted that the use of the valve is not limited to the intended position and orientation but may be deployed in any type of lumen or passageway. For example, although prosthetic heart valves are described herein as prosthetic aortic valves, those same or similar structures and features can be employed in other heart valves, such as the pulmonary valve, the mitral valve, or the tricuspid valve. Further, the term "proximal," when used in connection with a delivery device or system, refers to a position relatively close to the user of that device or system when it is being used as intended, while the term "distal" refers to a position relatively far from the user of the device. In other words, the leading end of a delivery device or system is positioned distal to the trailing end of the delivery device or system, when the delivery device is being used as intended. As used herein, the terms "substantially," "generally," "approximately," and "about" are intended to mean that slight deviations from absolute are included within the scope of the term so modified. As used herein, the prosthetic heart valves may assume an "expanded state" and a "collapsed state," which refer to the relative radial size of the stent.

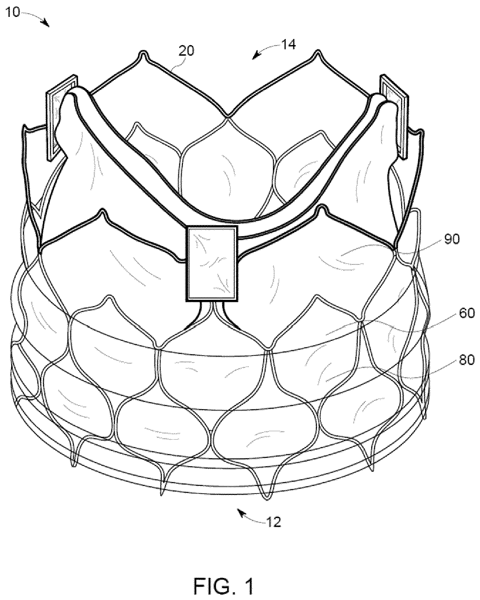

Fig. 1 is a perspective view of one example of a prosthetic heart valve 10. Prosthetic heart valve 10 may be a balloon-expandable prosthetic aortic valve, although in other examples it may be a self-expandable or mechanically-expandable prosthetic heart valve, intended for replacing a native aortic valve or another native heart valve. Prosthetic heart valve 10 is shown in an expanded condition in Fig. 1. Prosthetic heart valve 10 may extend between an inflow end 12 and an outflow end 14. Prosthetic heart valve 10 may include a collapsible and expandable frame 20, an inner cuff or skirt 60, an outer cuff or skirt 80, and a plurality of prosthetic leaflets 90. As should be clear below, prosthetic heart valve 10 is merely one example of a prosthetic heart valve, and other examples of prosthetic heart valves may be suitable for use with the concepts described below.

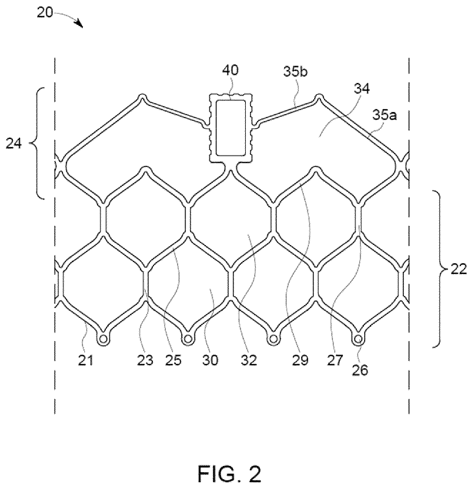

Fig. 2 is a front view of an example of a section of the frame 20 of prosthetic heart valve 10, as if cut longitudinally and laid flat on a table. The section of frame 20 in Fig. 2 may represent approximately one-third of a complete frame, particularly if frame 20 is used in conjunction with a three-leaflet prosthetic heart valve. In the illustrated example, frame 20 is a balloon-expandable stent and may be formed of stainless steel or cobalt-chromium, and which may include additional materials such as nickel and/or molybdenum. However, in some embodiments the stent may be formed of a shape memory material such as nitinol or the like. The frame 20, when provided as a balloon-expandable frame, is configured to collapse upon being crimped to a smaller diameter and/or expand upon being forced open, for example via a balloon within the frame expanding, and the frame will substantially maintain the shape to which it is modified when at rest.

Frame 20 may include an inflow section 22 and an outflow section 24. The inflow section 22 may also be referred to as the annulus section. In one example, the inflow section 22 includes a plurality of rows of generally hexagon-shaped cells. For example, the inflow section 22 may include an inflow-most row of hexagon-shaped cells 30 and an outflow-most row of hexagon-shaped cells 32. The inflow-most row of hexagonal cells 30 may be formed of a first circumferential row of angled or zig-zag struts 21, a second circumferential row of angled or zig-zag struts 25, and a plurality of axial struts 23 that connect the two rows. In other words, each inflow-most hexagonal cell 30 may be formed by two angled struts 21 that form an apex pointing in the inflow direction, two angled struts 25 that form an apex pointing in the outflow direction, and two axial struts that connect the two angled struts 21 to two corresponding angled struts 25. The outflow-most row of hexagonal cells 32 may be formed of the second circumferential row of angled or zig-zag struts 25, a third circumferential row of angled or zig-zag struts 29, and a plurality of axial struts 27 that connect the two rows. In other words, each outflow-most hexagonal cell 32 may be formed by two angled struts 25 that form an apex pointing in the inflow direction, two angled struts 29 that form an apex pointing in the outflow direction, and two axial struts that connect the two angled struts 27 to two corresponding angled struts 29. It should be understood that although the term "outflow-most" is used in connection with hexagonal cells 32, additional frame structure, described in more detail below, is still provided in the outflow direction relative to the outflow-most row of hexagonal cells 32.

In the illustrated embodiment, assuming that frame 20 is for use with a three-leaflet valve and thus the section shown in Fig. 2 represents about one-third of the frame 20, each row of cells 30, 32 includes twelve individual cells. However, it should be understood that more or fewer than twelve cells may be provided per row of cells. Further, the inflow or annulus section 22 may include more or fewer than two rows of cells. Still further, although cells 30, 32 are shown as being hexagonal, the some or all of the cells of the inflow section 22 may have other shapes, such as diamond-shaped, chevron-shaped, or other suitable shapes. In the illustrated embodiment, every cell 30 in the first row is structurally similar or identical to every other cell 30 in the first row, every cell 32 in the second row is structurally similar or identical to every other cell 32 in the second row, and every cell 30 in the first row is structurally similar or identical (excluding the aperture 26) to every cell 32 in the second row. However, in other examples, the cells in each row are not identical to every other cell in the same row or in other rows.

An inflow apex of each hexagonal cell 30 may include an aperture 26 formed therein, which may accept sutures or similar features which may help couple other elements, such as an inner cuff 60, outer cuff 80, and/or prosthetic leaflets 90, to the frame 20. However, in some examples, one or more or all of the apertures 26 may be omitted.

Still referring to Fig. 2, the outflow section 24 of the frame 20 may include larger cells 34 that have generally asymmetric shapes. For example, the lower or inflow part of the larger cells 34 may be defined by the two upper struts 29 of a cell 32, and one upper strut 29 of each of the two adjacent cells 32. In other words, the lower end of each larger cell 34 may be formed by a group of four consecutive upper struts 29 of three circumferentially adjacent cells 32. The tops of the larger cells 34 may each be defined by two linking struts 35a, 35b. The first linking strut 35a may couple to a top or outflow apex of a cell 32 and extend upwards at an angle toward a commissure attachment feature ("CAF") 40. The second linking strut 35b may extend from an end of the first linking strut 35a back downwardly at an angle and connect directly to the CAF 40. To the extent that the larger cells 34 include sides, a first side is defined by a portion of the CAF 40, and a second side is defined by the connection between first linking strut 35a and the corresponding upper strut 29 of the cell 32 attached to the first linking strut 35a.

The CAF 40 may generally serve as an attachment site for leaflet commissures (*e.g*. where two prosthetic leaflets 90 join each other) to be coupled to the frame 20. In the illustrated example, the CAF 40 is generally rectangular and has a longer axial length than circumferential width. The CAF 40 may define an interior open rectangular space. The struts that form CAF 40 may be generally smooth on the surface defining the open rectangular space, but some or all of the struts may have one or more suture notches on the opposite surfaces. For example, in the illustrated example, CAF 40 includes two side struts (on the longer side of the rectangle) and one top (or outflow) strut that all include alternating projections and notches on their exterior facing surfaces. These projections and notches may help maintain the position of one or more sutures that wrap around these struts. These sutures may directly couple the prosthetic leaflets 90 to the frame 20, and/or may directly couple an intermediate sheet of material (*e.g*. fabric or tissue) to the CAF 40, with the prosthetic leaflets 90 being directly coupled to that intermediate sheet of material. In some embodiments, tabs or ends of the prosthetic leaflets 90 may be pulled through the opening of the CAF 40, but in other embodiments the prosthetic leaflets 90 may remain mostly or entirely within the inner diameter of the frame 20. It should be understood that balloon-expandable frames are typically formed of metal or metal alloys that are very stiff, particularly in comparison to self-expanding frames. At least in part because of this stiffness, although the prosthetic leaflets 90 may be sutured or otherwise directly coupled to the frame at the CAFs 40, it may be preferable that most or all of the remaining portions of the prosthetic leaflets 90 are not attached directly to the frame 20, but are rather attached directly to an inner skirt 60, which in turn is directly connected to the frame 20. Further, it should be understood that other shapes and configurations of CAFs 40 may be appropriate. For example, various other suitable configurations of frames and CAFs are described in greater detail in U.S. Provisional Patent Application No. 63/579,378, filed August 29, 2023 and titled "TAVI Deployment Accuracy - Stent Frame Improvements," the disclosure of which is hereby incorporated by reference herein.

With the example described above, frame 20 includes two rows of hexagon-shaped cells 30, 32, and a single row of larger cells 34. In a three-leaflet embodiment of a prosthetic heart valve that incorporates frame 20, each row of hexagon-shaped cells 30, 32 includes twelve cells, while the row of larger cells includes six larger cells 34. As should be understood, the area defined by each individual cell 30, 32 is significantly smaller than the area defined by each larger cell 34 when the frame 20 is expanded. There is also significantly more structure (*e.g*. struts) that create each row of individual cells 30, 32 than structure that creates the row of larger cells 34.

One consequence of the above-described configuration is that the inflow section 22 has a higher cell density than the outflow section 24. In other words, the total numbers of cells, as well as the number of cells per row of cells, is greater in the inflow section 22 compared to the outflow section 24. The configuration of frame 20 described above may also result in the inflow section 22 being generally stiffer than the outflow section 24 and/or more radial force being required to expand the inflow section 22 compared to the outflow section 24, despite the fact that the frame 20 may be formed of the same metal or metal alloy throughout. This increased rigidity or stiffness of the inflow section 22 may assist with anchoring the frame 20, for example after balloon expansion, into the native heart valve annulus. The larger cells 34 in the outflow section 24 may assist in providing clearance to the coronary arteries after implantation of the prosthetic heart valve 10. For example, after implantation, one or more coronary ostia may be positioned above the frame 20, for example above the valley where two adjacent larger cells 34 meet (about halfway between a pair of circumferentially adjacent CAFs 40). Otherwise, one or more coronary ostia may be positioned in alignment with part of the large interior area of a larger cell 34 after implantation. Either way, blood flow to the coronary arteries is not obstructed, and a further procedure that utilizes the coronary arteries (*e.g.* coronary artery stenting) will not be obstructed by material of the frame 20. Still further, the lower rigidity of the frame 20 in the outflow section 24 may cause the outflow section 24 to preferentially foreshorten during expansion, with the inflow section 22 undergoing a relatively smaller amount of axial foreshortening. This may be desirable because, as the prosthetic heart valve 10 expands, the position of the inflow end of the frame 20 may remain substantially constant relative to the native valve annulus, which may make the deployment of the prosthetic heart valve 10 more precise. This may be, for example, because the inflow end of the frame 20 is typically used to gauge proper alignment with the native valve annulus prior to deployment, so axial movement of the inflow end of the frame 20 relative to the native valve annulus during deployment may make precise placement more difficult.

Referring back to Fig. 1, the prosthetic heart valve 10 may include an inner skirt 60 mounted to the interior surface of frame 20. The inner skirt 60 may be formed of tissue, such as pericardium, although other types of tissue may be suitable. In the illustrated example, the inner skirt 60 is formed of a woven synthetic fabric, such as polyethylene terephthalate ("PET") or polytetrafluoroethylene ("PTFE"), although other fabrics may be suitable, including fabrics other than woven fabrics. In some examples, the inner skirt 60 has straight or zig-zag shaped inflow and outflow ends that generally follow the contours of the cells 30, 32 of the inflow section 22 of frame 20. Preferably, inner skirt 60 is sutured to the frame 20 along the struts that form cells 30, 32. If apertures 26 are included, inner skirt 60 may also be coupled to frame 20 via sutures passing through apertures 26. Preferably, the inner skirt 60 does not cover (or does not cover significant portions of) the larger cells 34. The inner skirt 60 may be coupled to the frame 20 via mechanisms other than sutures, including for example ultrasonic welding or adhesives. Further, the inner skirt 60 may have shapes other than that shown, and need not have a zig-zag inflow or outflow end, and need not cover every cell in the inflow section 22. In fact, in some examples, the inner skirt 60 may be omitted entirely, with the outer skirt 80 (described in greater detail below) being the only skirt used with prosthetic heart valve 10. If the inner skirt 60 is provided, it may assist with sealing the prosthetic heart valve 10 within the heart, as well as serving as a mounting structure for the prosthetic leaflets 90 (described in greater detail below) within the frame 20.

Still referring to Fig. 1, the prosthetic heart valve 10 may include an outer skirt 60 mounted to the exterior surface of frame 20. The outer skirt 80 may be formed of tissue, such as pericardium, although other types of tissue may be suitable. In the illustrated example, the outer skirt 80 is formed of a woven synthetic fabric, such as PET or PTFE, although other fabrics may be suitable, including fabrics other than woven fabrics. In some examples, the outer skirt 80 has straight or zig-zag inflow end. Preferably, outer skirt 80 is sutured to the frame 20 and/or inner skirt 60 along the inflow edge of the outer skirt 80. If apertures 26 are included, outer skirt 80 may also be coupled to frame 20 via sutures passing through apertures 26. The outer skirt 80 may include a plurality of folds or pleats, such a circumferentially extending folds or pleats. The folds or pleats may be formed in the outer skirt 80 via heat setting, for example by placing the outer skirt 80 within a mold that forces the outer skirt 80 to form folds of pleats, and the outer skirt 80 may be treated with heat so that the outer skirt 80 tends to maintain folds or pleats in the absence of applied forces. The outflow edge of outer skirt 80 may be coupled to the frame 20 at selected, spaced apart locations around the circumference of the frame 20. In some embodiments, the outflow edge of outer skirt 80 may be connected to the inner skirt 60 along a substantially continuous suture line. Some or all of the outer skirt 80 between its inflow and outflow edges may remain not directly couples to the frame 20 or inner skirt 60. Preferably, the outer skirt 80 does not cover (or does not cover significant portions of) the larger cells 34. In use, the outer skirt 80 may directly contact the interior surface of the native heart valve annulus to assist with sealing, including sealing against PV leak. If folds or pleats are included with the outer skirt 80, the additional material of the folds or pleats may help further mitigate PV leak. However, it should be understood that the folds or pleats may be omitted from outer skirt 80, and the outer skirt 80 may have shapes other than that shown. In fact, in some examples, the outer skirt 80 may be omitted entirely, with the inner skirt 60 being the only skirt used with prosthetic heart valve 10. If the inner skirt 60 is omitted, the prosthetic leaflets 90 may be attached directly to the frame 20 and/or directly to the outer skirt 80.

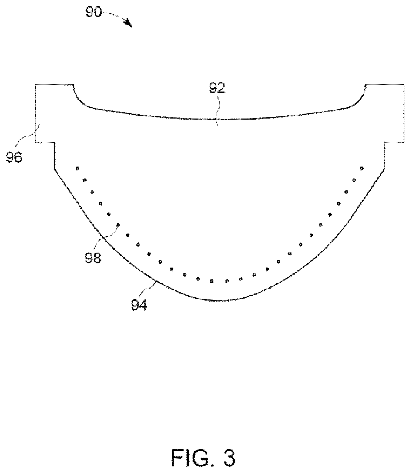

Fig. 3 is a front view of a prosthetic leaflet 90, as if laid flat on a table. In the illustrated example of prosthetic heart valve 10, a total of three prosthetic leaflets 90 are provided, although it should be understood that more or fewer than three prosthetic leaflets may be provided in other example of prosthetic heart valves. The prosthetic leaflet 90 may be formed of a synthetic material, such a polymer sheet or woven fabric, or a biological material, such a bovine or porcine pericardial tissue. However, other materials may be suitable. In on example, the prosthetic leaflet 90 is formed to have a concave free edge 92 configured to coapt with the free edges of the other leaflets to help provide the one-way valve functionality. The prosthetic leaflet 90 may include an attached edge 94 which is attached (e.g. via suturing) to other structures of the prosthetic heart valve 10. For example, the attached edge 94 may be coupled directly to the inner skirt 60, directly to the frame 20, and/or directly to the outer skirt 80. It may be preferable that the attached edge 94 is coupled directly only to the inner skirt 60, which may help reduce stresses on the prosthetic leaflet 90 compared to if the attached edge 94 were coupled directly to the frame 20. In some embodiments, a plurality of holes 98 may be formed along the attached edge 94 (or a spaced distance therefrom), for example via lasers. If included, the holes 98 may be used to receive sutures therethrough, which may make it easier to couple the prosthetic leaflet 90 to the inner skirt 60 during manufacturing. For example, the holes 98 may serve as guides if suturing is performed manually, and if the positions of the holes 98 are controlled via the use of layers, the holes 98 may be consistently placed among different prosthetic leaflets 90 to reduce variability between different prosthetic leaflets 90. Laflet tabs 96 may be provided at the junctions between the free edge 92 and the attached edge 94. Each leaflet tab 96 may be joined to a leaflet tab of an adjacent prosthetic leaflet to form prosthetic leaflet commissures, which may be coupled to the frame 20 via CAFs 40.

The prosthetic heart valve 10 may be delivered via any suitable transvascular route, for example transapically or transfemorally. Generally, transapical delivery utilizes a relatively stiff catheter that pierces the apex of the left ventricle through the chest of the patient, inflicting a relatively higher degree of trauma compared to transfemoral delivery. In a transfemoral delivery, a delivery device housing or supporting the valve is inserted through the femoral artery and advanced against the flow of blood to the left ventricle. In either method of delivery, the valve may first be collapsed over an expandable balloon while the expandable balloon is deflated. The balloon may be coupled to or disposed within a delivery system, which may transport the valve through the body and heart to reach the aortic valve, with the valve being disposed over the balloon (and, in some circumstances, under an overlying sheath). Upon arrival at or adjacent to the aortic valve, a surgeon or operator of the delivery system may align the prosthetic valve as desired within the native valve annulus while the prosthetic valve is collapsed over the balloon. When the desired alignment is achieved, the overlying sheath, if included, may be withdrawn (or advanced) to uncover the prosthetic valve, and the balloon may then be expanded causing the prosthetic valve to expand in the radial direction, with at least a portion of the prosthetic valve foreshortening in the axial direction.

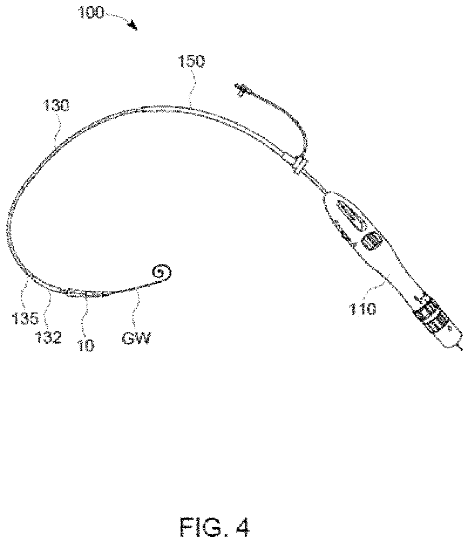

Fig. 4 illustrates one example of a delivery system 100, with the prosthetic heart valve 10 crimped over a balloon on a distal end of the delivery system 100. Although delivery system 100 and various components thereof are described below, it should be understood that delivery system 100 is merely one example of a balloon catheter that may be appropriate for use in delivering and deploying prosthetic heart valve 10.

In some examples, delivery system 100 includes a handle 110 and a delivery catheter 130 extending distally from the handle 110. An introducer 150 may be provided with the delivery system 100. Introducer 150 may be an integrated or captive introducer, although in other embodiments introducer 150 may be a non-integrated or non-captive introducer. In some examples, the introducer 150 may be an expandable introducer, including for example an introducer that expands locally as a large diameter components passes through the introducer, with the introducer returning to a smaller diameter once the large diameter components passes through the introducer. In other examples, the introducer 150 is a non-expandable introducer.

A guidewire GW may be provided that extends through the interior of all components of the delivery system 100, from the proximal end of the handle 110 through the atraumatic distal tip 138 of the delivery catheter 130. The guidewire GW may be introduced into the patient to the desired location, and the delivery system 100 may be introduced over the guidewire GW to help guide the delivery catheter 130 through the patient's vasculature over the guidewire GW.

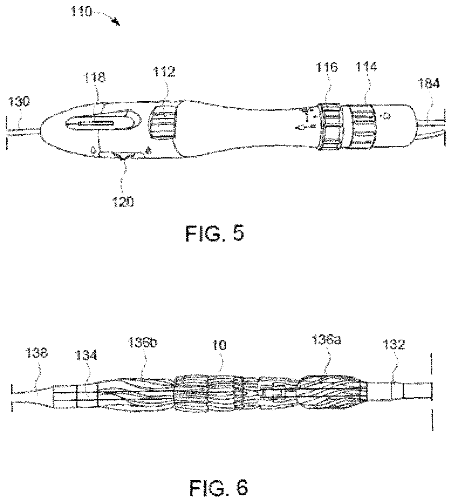

In some examples, the delivery catheter 130 is steerable. For example, one or more steering wires may extend through a wall of the delivery catheter 130, with one end of the steering wire coupled to a steering ring coupled to the delivery catheter 130, and another end of the steering wire operable coupled to a steering actuator on the handle 110. In such examples, as the steering actuator is actuated, the steering wire is tensioned or relaxed to cause deflection or straightening of the delivery catheter 130 to assist with steering the delivery catheter 130 to the desired position within the patient. For example, Fig. 5 is an enlarged view of the handle 110. Handle 110 may include a steering knob 112 that, upon rotation, tensions or relaxes the steering wires to deflect the distal end of the delivery catheter 130. A deflection indicator 118 may be included that shows the extent of the deflection of the distal end of delivery catheter 130.However, it should be understood that the steering functionality may be omitted in some examples, and in other examples steering actuators other than knobs may be utilized. Further, in some examples, including those shown in Figs. 6-7, the delivery catheter 130 includes an outer catheter 132, and an inner catheter 134. The steering functionality may be provided in either the outer catheter 132, or the inner catheter 134, or in both catheters.

Still referring to Figs. 4-5, the delivery system 100 may include additional functionality to assist with positioning the prosthetic heart valve 10. For example, in the illustrated example, handle 110 includes a commissure alignment actuator 114, which may be positioned near a proximal end of the handle or at any other desired location. In the illustrated example, the commissure alignment actuator 114 is in the form of a rotatable knob, although other forms may be suitable. The commissure alignment knob 114 may be rotationally coupled to a portion of the delivery catheter 130 supporting the prosthetic heart valve 10. For example, the commissure alignment actuator 114 may be rotationally coupled to an inner catheter 134 which supports the prosthetic heart valve 10 in the crimped condition. With this configuration, rotating the commissure alignment knob 114 may cause the inner catheter 134 to rotate about its longitudinal axis, and thus cause the prosthetic heart valve 10 to rotate about its longitudinal axis. If a commissure alignment actuator 114 is included, it may be used to help ensure that, upon deployment of the prosthetic heart valve 10 into the native valve annulus, the commissures of the prosthetic heart valve are in rotational alignment with respective ones of the native valve commissures (*e.g*. within +/- 2.5 degrees of rotational alignment, within +/- 5 degrees of rotational alignment, within +/- 10 degrees of rotational alignment, within +/- 15 degrees of rotational alignment, *etc.*)*.* Although commissure alignment actuator 114 is shown in this example as a knob positioned at or near a proximal end of the handle 110, it should be understood that the actuator 114 may take forms other than a knob, may be positioned at other suitable locations, and may be omitted entirely if desired.

Still referring to Figs. 4-5, the delivery system 100 may include even further functionality to assist with positioning the prosthetic heart valve 10. For example, in the illustrated example, handle 110 includes an axial alignment actuator 116, which may be positioned near a proximal end of the handle, including distal to the commissure alignment actuator 114, or at any other desired location. In the illustrated example, the axial alignment actuator 116 is in the form of a rotatable knob, although other forms may be suitable. The axial alignment knob 116 may be operably coupled to a portion of the delivery catheter 130 supporting the prosthetic heart valve 10. For example, the axial alignment actuator 116 may include internal threads that engage external threads of a carriage that is coupled to an inner catheter 134 which supports the prosthetic heart valve 10 in the crimped condition. In such an example, the carriage may be rotatably fixed to the handle 110. With this configuration, rotating the axial alignment knob 116 may cause the carriage to advance distally or retract proximally as the inner threads of the axial alignment knob 116 mesh with the external threads of the carriage, but the carriage is prevented from rotating. As the carriage advances distally or retracts proximally, the inner catheter 134 may correspondingly advance distally or retract proximally, and thus cause the prosthetic heart valve 10 to advanced distally or retract proximally. It should be understood that, if axial alignment actuator 116 is included, it have a small total range of motion. In other words, the rough or coarse axial alignment between the prosthetic heart valve 10 and native valve annulus may be achieved by physically advancing the entire delivery catheter 130 by pushing it through the vasculature while holding the handle 110. However, for fine and more controlled adjustment of the axial position of the prosthetic heart valve 10 relative to the native valve annulus, which may be performed just prior to or during deployment of the prosthetic heart valve 10, the axial alignment knob 116 may be used. If an axial alignment actuator 116 is included, it may be used to help ensure that, upon deployment of the prosthetic heart valve 10 into the native valve annulus, the inflow end of the of the prosthetic heart valve is in axial alignment with the inflow aspect of the native valve annulus (e.g. within +/- 0.5mm of axial alignment, within +/- 1.0mm of axial alignment, within +/- 1.5mm of axial alignment, within +/- 2.0 mm of axial alignment, e*tc.*)*.* Although axial alignment actuator 116 is shown in this example as a knob positioned at or near a proximal end of the handle 110, it should be understood that the actuator 116 may take forms other than a knob, may be positioned at other suitable locations, and may be omitted entirely if desired.

In addition to steering and positioning actuators, delivery system 100 may include a balloon actuator 120. In the illustrated example, balloon actuator 120 is positioned on the handle 110 near a distal end thereof, and is provided in the form of a switch. Balloon actuator 120 may be actuated to cause inflation or deflation of a balloon 136 that is part of the delivery system 100. For example, referring briefly to Figs. 6-7, the delivery system 100 may include a balloon 136 that overlies a distal end of inner catheter 134 and which receives the prosthetic heart valve 10 in a crimped condition thereon. In the example illustrated in Fig. 6, the balloon 136 includes a proximal pillowed portion 136a, a distal pillowed portion 136b, and a central portion over which the prosthetic heart valve 10 is crimped. The proximal pillow 136a and the distal pillow 136b may form shoulders on each side of the prosthetic heart valve 10, which may help ensure the prosthetic heart valve 10 does not move axially relative to the balloon 136 and/or inner catheter 134 during delivery. The shoulder formed by the distal pillow 136 may also help protect the inflow edge of the prosthetic heart valve 10 from contact with the anatomy during delivery. For example, during a transfemoral delivery, as the distal end of the delivery catheter 130 traverse the sharp bends of the aortic arch (or during initial introduction into the patient), there is a relatively high likelihood the inflow end of the prosthetic heart valve 10 (which is the leading edge during transfemoral delivery) will contact a vessel wall (or a components of an introduction system) causing dislodgment of the prosthetic heart valve 10 relative to the balloon 136. The distal pillow 136 may tend to have an equal or larger outer diameter than the inflow end of the prosthetic heart valve 10 (when the prosthetic heart valve 10 is crimped and the balloon 136 is deflated), which may help ensure the inflow edge of the prosthetic heart valve 10 does not inadvertently contact another structure during delivery. In some examples, the pillowed portions 136a, 136b may be formed via heat setting. Additional related features for use in similar balloon catheter delivery systems are described in greater detail in U.S. Provisional Patent Application No. 63/382,812, filed November 8, 2022 and titled "Prosthetic Heart Valve Delivery and Trackability," the disclosure of which is hereby incorporated by reference herein.

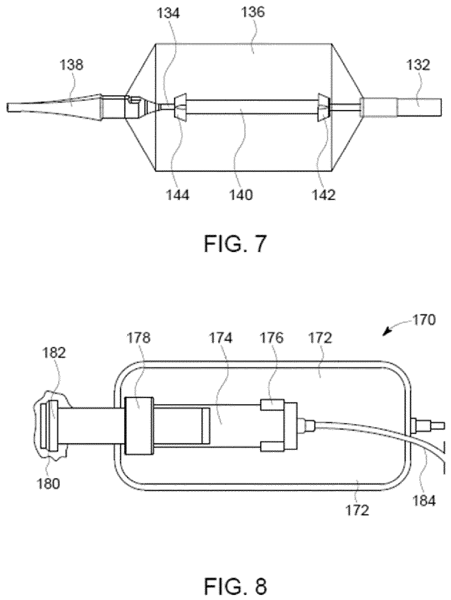

In order to deploy the prosthetic heart valve 10, the balloon 136 is inflated, for example by actuating the balloon actuator 120 to force fluid (such as saline, although other fluids, including liquids or gases, could be used) into the balloon 136 to cause it to expand, causing the prosthetic heart valve 10 to expand in the process. For example, the balloon actuator 120 may be pressed forward or distally to cause fluid to travel through an inflation lumen within delivery catheter 130 to inflate the balloon 136. Fig. 7 illustrates an example of the balloon 136 after being inflated, with the prosthetic heart valve 10 omitted from the figure for clarity. In the illustrated example, the balloon 136 may be formed to have a distal end that is fixed to a portion of an atraumatic distal tip 138. The distal tip 138 may be tapered to help the delivery catheter 130 move through the patient's vasculature more smoothly. A proximal end of the balloon 136 may be fixed to a distal end of outer catheter 132. The inflation lumen may be the space between the outer catheter 132 and the inner catheter 134, or in other embodiments may be provided in a wall of the inner catheter 134, or in any other location that fluidly connects the interior of the balloon 136 to a fluid source outside of the patient that is operable coupled to the delivery system 100.

Referring to Fig. 7, in some examples, a mounting shaft 140 may be provided on the inner catheter 134. A proximal stop 142 and/or a distal stop 144 may be provided, for example at opposite ends of the mounting shaft 140. If the mounting shaft 140 is included, it may provide a location on which the prosthetic heart valve 10 may be crimped. If the proximal stop 142 and/or distal stop 144 is provided, they may provide physical barriers to the prosthetic heart valve 10 moving axially relative to the balloon 136. In one example, the proximal stop 142 may taper from a larger distal diameter to a smaller proximal diameter, and the distal stop may taper from a larger proximal diameter to a smaller distal diameter. The spacing between the proximal stop 142 and the distal stop 144, if both are included, may be slightly larger than the length of the prosthetic heart valve 10 when it is crimped over mounting shaft 140. However, it should be understood that one or both of the stops 142, 144 may be omitted, and the mounting shaft 140 may also be omitted. If the mounting shaft 140 is included, it is preferably axially and rotationally fixed to the inner catheter 134 so that movement of the inner catheter 134 causes corresponding movement of the mounting member 140, and thus the prosthetic heart valve 10 when mounted thereon.

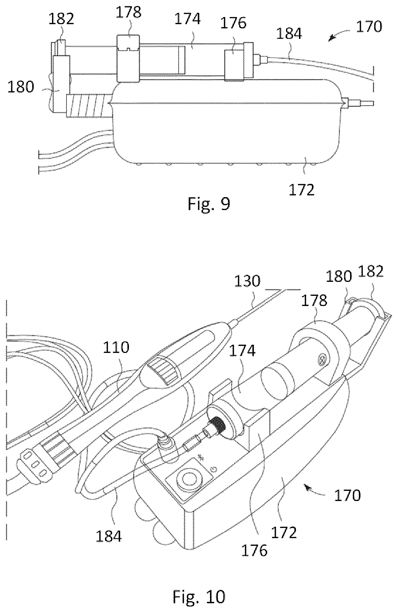

Before describing the use of balloon actuator 120 in more detail, it should be understood that in some embodiments, the balloon actuator 120 may be omitted and instead a manual device, such as a manual syringe, may be provided along with delivery system 100 in order to manually push fluid into balloon 136 during deployment of the prosthetic heart valve 10. However, in the illustrated example of delivery system 100, the balloon actuator 120 provides for a motorized and/or automated (or semi-automated) balloon inflation functionality. For example, Fig. 8 and Fig. 9 illustrate an example of a balloon inflation system 170. Balloon inflation system 170 may include a housing 172 that houses one or more components, which may include a motor, one or more batteries, electronics for control and/or communication with other components, *etc.* Housing 172 may include one or more fixed cradles to receive a syringe 174. In the illustrated embodiment, a distal cradle 176 is provide with an open "C"- or "U"-shaped configuration so that the distal end of the syringe 174 may be snapped into or out of the distal cradle 176. A proximal cradle 178 may also be provided, which may have a "C"- or "U"-shaped bottom portion hingedly connected to a "C"- or "U"-shaped top portion. This configuration may allow for the proximal end of the outer body of the syringe 174 to be snapped into the bottom portion of proximal cradle 178, and the top portion of proximal cradle 178 may be closed and connected to the bottom portion to fully circumscribe the outer body of the syringe 174 to lock the syringe 174 to the housing 172. It should be understood that more or fewer cradles, of similar or different designs, may be included with housing 172 to help secure the syringe 174 to the housing 172 in any suitable fashion.

The balloon inflation system 170 may include a moving member 180. In the illustrated embodiment, moving member 180 includes a "C"- or "U"-shaped cradle to receive a plunger handle 182 of the syringe 174 therein, the cradle being attached to a carriage that extends at least partially into the housing 172. The carriage of the moving member 180 may be generally cylindrical, and may include internal threading that mates with external threading of a screw mechanism (not shown) within the housing 172 that is operably coupled to a motor. In some embodiments, the carriage may have the general shape of a "U"-beam with the flat face oriented toward the top. The moving member 180 may be rotationally fixed to the housing 172 via any desirable mechanism, so that upon rotation of the screw mechanism by the motor, the moving member 180 advances farther into the housing 172, or retracts farther away from the housing 172, depending on the direction of rotation of the screw mechanism. While the plunger handle 182 is coupled to the moving member 180, advancement of the moving member 180 forces fluid from the syringe 174 toward the balloon 136, while retraction of the moving member 180 withdraws fluid from the balloon 136 toward the syringe 174. It should be understood that the motor, or other driving mechanism, may be located in or outside the housing 172, and any other suitable mechanism may be used to operably couple the motor or other driving mechanism to the moving member 180 to allow for axial driving of the plunger handle 182.

As shown in each of Fig. 8, Fig. 9, and Fig. 10, the distal end of syringe 174 may be coupled to tubing 184 that is in fluid communication with an inflation lumen of delivery catheter 130 that leads to the balloon 136 at or near the distal end of the delivery system 100. Tubing 184 may allow for the passage of the fluid (*e.g.,* saline) from the syringe 174 toward the balloon 136, or for withdrawal of fluid from the balloon 136 toward the syringe 174, for example based on whether the balloon actuator 120 is pressed forward or backward.

Although not separately numbered in Fig. 8, Fig. 9, and Fig. 10, the housing 172 may include one or more cables extending from the housing, for example to allow for transmission of power (*e.g*. from AC mains or another component with which the cable is coupled) and/or transmission of data, information, control commands, *etc.* For example, one cable may couple the housing 172 to handle 110 so that controls on the handle 110 (*e.g*. balloon actuator 120) may be used to activate the balloon inflation system 170 in the desired fashion. Another cable may couple to a computer display or similar device to provide information regarding the inflation of the balloon 136. However, it should be understood that any transmission of data or information may be provided wirelessly instead of via a wired connection, for example via a Bluetooth or other suitable connection. Additional and related features of balloon inflation system 170, related systems, and the uses thereof are described in U.S. Patent Application No. 18/311,458, the disclosure of which is hereby incorporated by reference herein.

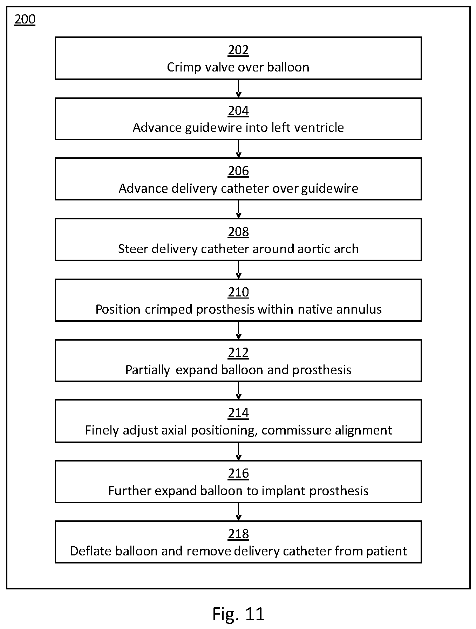

Fig. 11 is a flowchart showing exemplary steps in an implantation procedure 200 to implant the prosthetic heart valve 10 of Fig. 1 into a patient using the delivery system 100 of Fig. 4. However, it should be understood that not all of the steps shown in connection with implantation procedure 200 need to be performed, and various steps not explicitly shown and described in connection with procedure 200 may be performed as part of the implantation procedure. At the beginning of the procedure 200 in step 202, the prosthetic heart valve 10 may be collapsed over or crimped onto balloon 136, with the balloon 136 being mostly or entirely deflated after the crimping procedure. It should be understood that crimping step 202 may be performed at any time prior to the procedure, including at the beginning of the procedure, or at an earlier stage before the delivery system 100 is provided to the end user. In other words, the crimping step 202 may be performed during a manufacturing stage of the delivery system 100 and/or prosthetic heart valve 10. During an early stage of the implantation procedure 200, a guidewire GW may be advanced into the patient in step 204, for example via the femoral artery, around the aortic arch, through the native aortic valve, and into the left ventricle. The guidewire GW may be used as a rail for other devices that need to access this pathway. For example, in step 206, the atraumatic distal tip 138 may be advanced over the proximal end of the guidewire GW, and the delivery catheter 130 may be advanced over guidewire GW toward the native aortic valve. During this initial advancement of the delivery catheter 130 into the patient, the introducer 150 (if included) may be positioned distally, for example so that it covers the prosthetic heart valve 10 or so that it is positioned just proximal to the prosthetic heart valve 10. Advancement of the delivery catheter 130 and introducer 150 may continue until a proximal hub of the introducer is in contact with the patient's skin (or in contact with another device that enters the patient's femoral artery. At this point, the introducer 150 may stop moving axially relative to the patient, with the delivery catheter 130 continuing to advance relative to the introducer 150. If steering capability is provided, the delivery catheter 130 may be steered or deflected at any point to assist with achieving the desired pathway of the delivery catheter 130. As on example, in step 208, the steering knob 112 may be actuated to deflect the distal end of the delivery catheter 130 as it traverses the sharp bends of the aortic arch. Advancement of the delivery catheter 130 may continue in step 210 until the prosthetic heart valve 10, while still crimped or collapsed, is positioned within the native aortic valve annulus. With the desired position achieved, the balloon 136 may be partially inflated, for example by pressing balloon actuator 120 forward, to partially expand the prosthetic heart valve 10 in step 212. In some examples, it is desirable to expand the prosthetic heart valve 10 only partially in step 212, because the position of the prosthetic heart valve 10 (including rotational and/or axial positioning) relative to the native aortic valve annulus may shift during this partial expansion. After the partial expansion of step 212, the user may examine the positioning of the prosthetic heart valve 10 relative to the native aortic valve annulus. If desired, in step 214, the axial positioning of the partially-expanded prosthetic heart valve 10 relative to the native aortic valve annulus may be finely adjusted (*e.g.* by actuating axial alignment actuator 116) and/or the rotational orientation of the prosthetic heart valve 10 relative to the native aortic valve may be finely adjust (*e.g.* by actuating commissure alignment actuator 114). When the desired axial alignment is achieve and the desired rotational alignment (*e.g.* rotational alignment between the prosthetic commissure and the native commissures) is achieved, the balloon 136 may be fully expanded in step 216 to fully expand the prosthetic heart valve 10 and to anchor the prosthetic heart valve 10 in the native aortic valve annulus in the desired position and orientation. After deployment is complete, the balloon 136 may be deflated in step 218, for example by pressing actuating balloon 120 backward, and the delivery catheter 130 and guidewire GW may be removed from the patient to complete the procedure. It should be understood that the nine steps shown in Fig. 11 as part of procedure 200 are merely exemplary of a single example of an implantation procedure, and steps shown may be omitted, steps not shown may be included, and steps may be provided in any order deemed appropriate by the physician and/or medical personnel.

Although various components of a prosthetic heart valve 10 and delivery system 100 are described above, it should be understood that these components are merely intended to provide better context to the systems, features, and/or methods described below. Thus, various components of the systems described above may be modified or omitted as appropriate without affecting the systems, features, and/or methods described below. For example, prosthetic heart valves other than the specific configuration shown and described in connection with Figs. 1-3 may be used with delivery systems other than the specific configuration shown and described in connection with Figs. 4-10 as part of an implantation procedure that uses steps other than the specific configuration shown and described in connection with Fig. 11, without affecting the inventive systems, features, and/or methods described below.

There may be durability concerns when tissue is used for a component of a prosthetic heart valve (e.g., leaflets, inner cuff, and/or outer cuff), particularly with valves that are older than 10 to 15 years. In some examples, certain portions of a prosthetic heart valve may include alternative non-tissue materials (e.g., fabrics, polymers and/or metals or combinations thereof), and this may represent an opportunity to improve the durability of a surgical or transcatheter (e.g., self-expanding or balloon expanding) heart valve replacement by eliminating or reducing the degradation of the tissue (e.g., via thrombus, HALT, calcification, etc.). While alternative leaflet materials have been extensively researched in the past, common issues related to handleability, tearing, thrombosis, calcification, etc. have been observed. Additionally, bioactive materials (i.e., those allowing for cellular infiltration) have also been challenged during the healing response by alpha SMA cells. Specifically, for alternative materials, α-SMA cells are known to have adverse effects (e.g., by causing leaflet retraction) *in vivo.* Therefore, the present disclosure outlines certain methods and processes to reduce and/or eliminate cell attachment to the alternative non-tissue material and/or modify the cells such that they are non-contractile. In some examples modifying the cellular response to a component may be achieved via physical (e.g., contact guidance) or chemical (e.g., changing the cellular phenotype) methods.

Sputtering enables a very thin coating to be applied on a substrate, which may limit the deleterious impact on device performance that is often associated with coatings. This process coats the substrate with a thin layer of the deposited material which does not impede mechanical function (i.e., flexibility) of the substrate, while modifying the surface properties to mitigate cell attachment or modify the cell properties. In this process, a target material, such as copper or platinum, is bombarded with high-energy ions in a vacuum chamber, causing atoms to be ejected and deposited onto the component (e.g., a leaflet). This coating may improve biocompatibility, corrosion resistance and/or durability, ensuring the valve performs reliably within the human body. The precise control over coating thickness and uniformity makes sputter coating ideal for sensitive and complex medical components.

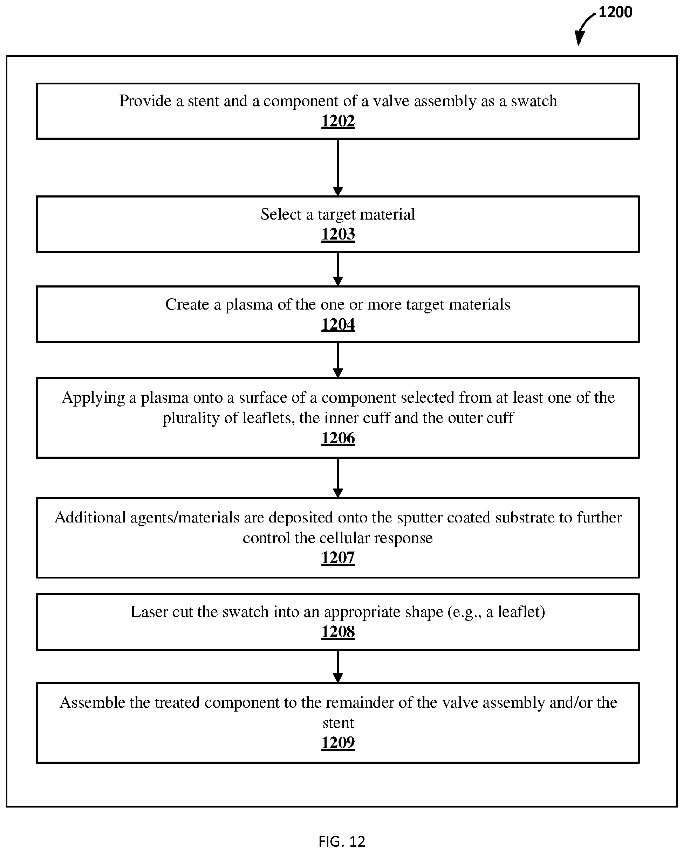

As shown in Fig. 12, in some examples, a valve component may undergo a sputter coating process 1200. The process 1200 may begin by providing a stent and the various element of a valve assembly (step 1202). The valve assembly may include one or more cuffs (e.g., an inner cuff and/or an outer cuff), and one or more leaflets (e.g., two leaflets or three leaflets). In some examples, components of the valve assembly may include tissue and/or non-tissue materials that are provided as generic swatches (e.g., rectangular segments or pieces that are not yet suitable for assembly). In some examples, the non-tissue swatch component to be sputter coated may include a fabric substrate that is woven or knit, and/or may comprise ultra-high molecular weight polyethylene (UHMWPE) or polyethylene terephthalate (PET). Alternative substrates may also include single-layer extruded, molded or formed polymers, or multi-layer composite polymers. After selecting the appropriate swatch components, the sputter coating process 1200 aims to modify the surface of a component by attaching one or more target materials to the component. The target material may be chosen or selected based on the intended result (step 1203). Target materials that could be sputter coated to the surface of a component or substrate may include, but are not limited to, any one or more of precious and non-precious metals, pure elements, compounds and/or alloys. For example, a target material may include a metal (e.g., transition metals or metallic alloys) such as zinc, magnesium, gold, palladium, stainless steel, or vanadium, molybdenum, and cobalt containing compounds, copper, titanium, silver, aluminum, tungsten, nickel, tin and/or platinum, non-metals such as carbon, silicon and/or graphite, metal-oxides such as titanium dioxide (TiO₂), iron oxide (Fe₂O₃), tungsten trioxide (WO₃), and/ non-metal oxides such as silicon dioxide (SiO₂), and/or ceramics. Other examples of target materials may include silver oxide (Ag₂O), copper oxide (CuO) and aluminum oxide (Al₂O₃). In some examples, sputter coating involves creating a plasma of the target material to be deposited, then bonding it to the surface of the component by bombarding the substrate material with gaseous ions to create a film on the substrate (step 1204).

One or more of the components of the valve assembly may be chosen and the sputter coating process 1200 may include sputter coating a thin layer (e.g., between 1 nm - 10µm) of the one or more target materials to the surface of a component to modify material surface properties which will reduce or inhibit cell attachment to the surface and/or modify the attaching cells to mitigate the leaflet retraction observed *in vivo* (step 1206). Optionally, additional agents/materials can be deposited onto the sputter coated substrate that further control the cellular response (step 1207). Such additional agents/materials may be deposited, before, during or after the sputter coating process. In some embodiments, the agent/materials are added before and after the sputter coating process. In some examples, sputter coating includes applying the one or more target materials on some or all surfaces of the component (e.g., on both sides of a leaflet). It will be understood that, when used in this context, the term "component" refers to any one or more of a leaflet, an inner cuff and an outer cuff. That is, the sputter coating process may be applied to any one of these components, to all of them, or to any combination, and the sputter coating process may be carried out separately for each component, or all components may undergo the sputter coating process simultaneously. The sputter coating process may also be carried out on a portion of a component (e.g., on known areas of a component that are in close proximity or in contact with, the adjacent myocardium to create a barrier/shield so that cells do not migrate across. After applying the plasma, the swatches (e.g., leaflet swatches, cuff swatches, etc.) may be laser cut to a suitable shape (step 1208). It will be understood, however, that in an alternative method, the component(s) may instead be first laser cut then treated according to this process. That is, the various components of the valve assembly may be initially provided in the shapes necessary for assembly.

After applying the plasma (and cutting the shapes, if needed), the process may include assembling the treated component to the remainder of the valve assembly and/or the stent (step 1209). It will be understood that, in other examples, the order of operation may be modified, and the plasma may be applied prior to or after attachment of the component to the fully or partially assembled prosthetic heart valve. Additionally, as noted, the sputter coating process could be applied to the entirety of a component, or to portions of each component (e.g., all of the leaflet(s), or to only portion of cuff only, or to only the inner surface of components, etc.) depending on the intended result of the sputter coating process.

As noted above with reference to step 1207, additional agents/materials may be deposited onto the sputter coated substrate to further control the cellular response. While the sputter coating material itself may modulate the cellular response, conjugating a molecule/material to it may also be advantageous. These molecules/materials may be selectively chosen to mitigate cell attachment, which may be accomplished in a variety of auxiliary or secondary techniques. In some examples, the auxiliary or secondary techniques may include contact guidance, which is the physical interaction of the cells with the substrate. In some examples, the methods to limit attachment may include one or more of the following methods/procedures: providing very smooth surfaces, peptide sequences, hydrophobicity of the material, material composition and/or chemical agents (e.g., drugs). In some examples, the auxiliary or secondary technique may include modulating the cell behavior (i.e., phenotype) that prevent the cells from spreading (i.e., fully attaching) or pulling on the substrate (via alpha-SMA expression) with certain molecules. These molecules may include drugs (e.g., everolimus) or chemical agents (e.g., glutaraldehyde).

Although certain examples have been provided, it will be understood that these are merely exemplary and not limiting. For example, though the preceding disclosure has mainly been directed to balloon-expandable valves, the principles discussed may also be applied to other types of prosthetic heart valve (e.g., surgical valves and self-expandable valves) or other implantable medical devices. Additionally, multiple locations within a prosthetic valve can be envisioned for sputter coating, either on the entire device or to select components, or to select regions within particular components. Modification may be directed to certain main region(s) of the device where cellular attachment is not wanted (e.g., on the leaflet or cuff). For example, the process may be applied to only the non-leaflet portions of the device, which may be advantageous if the sputter coating and subsequent conjugation adversely impacts leaflet motion and/or durability. Secondary or auxiliary techniques may also be applied, such as chemical substances to alter the cellular phenotype, and the same or different secondary techniques may be applied to different components. Moreover, the steps in the described methods are merely exemplary and may be modified, repeated, or taken out of sequence. Any of the steps in the method may also be optional. For example, a component may be treated prior to vapor deposition/sputtering. In some examples, a fabric may be plasma etched to reduce the surface free energy prior to applying the sputter coating in step 1206 and the method may, thereafter, continue with step 1207. Likewise, the laser cutting of step 1208 may occur prior to selecting a target material in step 1203. Additionally, it will be understood that the principles may be applied to transcatheter mitral valve replacement, and other implantable medical devices.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A method of manufacturing a prosthetic heart valve comprising:
providing a stent and a valve assembly, the valve assembly including at least one of an inner cuff and an outer cuff, and a plurality of leaflets;
sputter coating a layer of one or more target materials onto a surface of at least one component selected from the plurality of leaflets, the inner cuff and the outer cuff; and
coupling the valve assembly to the stent.

2. The method of claim 1, wherein sputter coating comprises creating a plasma of the one or more target materials and depositing the plasma onto the surface of the component.

3. The method of claim 1 or 2, wherein sputter coating comprises sputter coating the plurality of leaflets.

4. The method of any of claims 1-3, wherein sputter coating comprises sputter coating at least one of the inner cuff and the outer cuff.

5. The method of any of claims 1-4, further comprising the step of laser cutting the component to a desired shape after the sputter coating step.

6. The method of any of claims 1-5, wherein sputter coating comprises sputter coating the layer of the one or more target materials onto an entire surface of the component.

7. The method of any of claims 1-5, wherein sputter coating comprises sputter coating the layer of the one or more target materials on only a portion of the surface of the component.

8. The method of any of claims 1-7, wherein the one or more target materials comprises at least one of a metal, a non-metal, a metal oxide and a non-metal oxide.

9. The method of any of claims 1-8, wherein the one or more target materials comprises at least one of Cu, Ti, Ag, Al, W, Ni, Sn and Pt.

10. The method of any of claims 1-9, wherein the one or more target materials comprises at least one of C, Si and graphite.

11. The method of any of claims 1-10, wherein the one or more target materials comprises at least one of TiO₂, Fe₂O₃ and WO₃.

12. The method of any of claims 1-11, wherein the one or more target materials comprises at least one of SiO₂ and ceramics.

13. The method of any of claims 1-12, further comprising adding a secondary agent to the component.

14. The method of claim 13, wherein adding the secondary agent comprises contact guidance or modulating cell behavior by applying the secondary agent.

15. The method of claim 13 or 14, wherein the secondary agent comprises a drug, a chemical agent, or glutaraldehyde.
